(19) Europäisches Patentamt
European Patent Office
Office européen des brevets

(11) Veröffentlichungsnummer : **0 101 811**
**B1**

(12) # EUROPÄISCHE PATENTSCHRIFT

(45) Veröffentlichungstag der Patentschrift : **18.09.85**

(51) Int. Cl.[4] : **C 07 C175/00**, B 01 J 31/12

(21) Anmeldenummer : **83105997.7**

(22) Anmeldetag : **20.06.83**

(54) **Oxidation von Retroionon.**

(30) Priorität : **20.07.82 CH 4424/82**

(43) Veröffentlichungstag der Anmeldung : **07.03.84 Patentblatt 84/10**

(45) Bekanntmachung des Hinweises auf die Patenterteilung : **18.09.85 Patentblatt 85/38**

(84) Benannte Vertragsstaaten : **AT BE CH DE FR GB IT LI NL**

(56) Entgegenhaltungen :
**EP-A- 0 036 651**
**US-A- 4 098 827**
**Chemical Abstracts Band 77, Nr. 17, 23. Oktober 1972, Columbus, Ohio, USA K. INA et al. "Photooxygenation of ionones. II. Photooxygenation of alpha-ionone", Seite 403, Spalte 2, Abstract Nr. 113856s**
**Chemical Abstracts Band 84, Nr. 9. 1. März 1976, Columbus, Ohio, USA D.L. DAVIS et al. "Chemistry of tobacco constituents. Oxidation of alpha-ionone and the acid-catalyzed rearrangement of 5-keto-alpha-ionone", Seite 294, Spalte 2, Abstract Nr. 56678d**

(73) Patentinhaber : **F. HOFFMANN-LA ROCHE & CO. Aktiengesellschaft**
**CH-4002 Basel (CH)**

(72) Erfinder : **Lohri, Bruno, Dr.**
**Schwarzackerstrasse 53**
**CH-4303 Kaiseraugst (CH)**

(74) Vertreter : **Cottong, Norbert A. et al**
**Grenzacherstrasse 124 Postfach 3255**
**CH-4002 Basel (CH)**

**EP 0 101 811 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von 4-Oxo-β-ionon, einem wertvollen Zwischenprodukt in Carotinoidsynthesen.

Erfindungsgemäss wird 4-Oxo-β-ionon der Formel

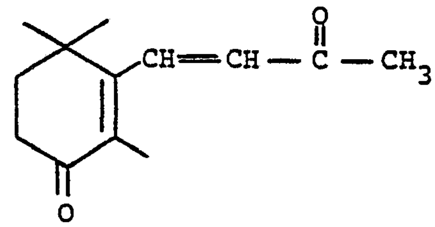

(I)

dadurch hergestellt, dass man Retroionon der Formel

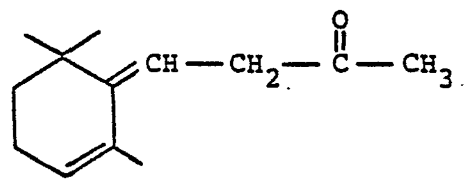

(II)

mit Sauerstoff oder einem sauerstoffhaltigen Gas oxidiert.

4-Oxo-β-ionon wurde bisher im allgemeinen aux β-lonon durch Oxidation mit tert.-Butylchromat oder Natriumchlorat hergestellt. Die Oxidation mit tert.-Butylchromat ist jedoch zur Herstellung in grösserem Massstab schlecht geeignet, während bei der Oxidation mit Natriumchlorat grosse Mengen an Oxidationsmittel benötigt und nur relativ geringe Ausbeuten erreicht werden.

Ferner wurde bereits β-lonon mit Luft oder Sauerstoff oxidiert. Diese Methode ergibt jedoch im allgemeinen komplexe Gemische und ist deshalb zur Herstellung von 4-Oxo-β-ionon ebenfalls schlecht geeignet.

Es wurde nun überraschenderweise gefunden, dass 4-Oxo-β-ionon auf einfache und billige Weise und in guter Ausbeute durch Oxidation des leicht zugänglichen Retroionon mit Sauerstoff oder einem sauerstoffhaltigen Gas erhalten werden kann.

Das erfindungsgemässe Verfahren kann mit oder ohne Anwesenheit eines Katalysators durchgeführt werden. Geeignete Katalysatoren sind beispielsweise die $C_1$-$C_{20}$-Carboxylate und die $C_2$-$C_{20}$-Enolate von Uebergangsmetallen, wie z. B. Palladium, Chrom, Kobalt, Nickel, Lanthan, Molybdän oder Vanadium. Bevorzugte Carboxylate sind die Acetate, und bevorzugte Enolate sind die Acetylacetonate. Vorzugsweise wird die Oxidation in Gegenwart eines Acetates oder insbesondere eines Acetylacetonates von Vanadium, Chrom, Kobalt, Nickel oder Molybdän durchgeführt. Besonders gute Ergebnisse werden mit Vanadylacetylacetonat oder Vanadium-(III)-acetylacetonat erzielt.

Das erfindungsgemässe Verfahren kann mit oder ohne Zusatz einer Base durchgeführt werden. Geeignete Basen sind beispielsweise Pyridin und alkylierte Pyridine. Bevorzugte Base ist 2-Methyl-5-äthylpyridin. Der Zusatz von Pyridin oder einem alkylierten Pyridin hat sich vor allem in den Fällen als vorteilhaft erwiesen, in denen als Katalysator ein Acetat oder Acetylacetonat von Vanadium, Chrom, Kobalt, Nickel oder Molybdän verwendet wird. Die Base kann auch zugleich als Lösungsmittel dienen.

Das erfindungsgemässe Verfahren kann mit oder ohne inertem organischem Lösungsmittel durchgeführt werden. Geeignete Lösungsmittel sind beispielsweise chlorierte Alkane, Carbonsäureamide, Hydroxyäther, chlorierte aromatische Kohlenwasserstoffe, Sulfoxide, Alkanole und Aether, wie Aethylenchlorid, Chloroform, Dimethylformamid, Acetamid, Methyldiglykol, Methylcellosolve, Chlorbenzol, Dimethylsulfoxid, tert.-Butanol, Monoglym und dergleichen. Eine gute Selektivität der Oxidation wird vor allem bei Verwendung von Carbonsäureamiden, Hydroxyäthern, chlorierten aromatischen Kohlenwasserstoffen und insbesondere chlorierten Alkanen erhalten. Besonders bevorzugt ist die Verwendung von Aethylenchlorid.

Auf Grund des oben Gesagten wird die erfindungsgemässe Oxidation von Retroionon besonders bevorzugt in Aethylenchlorid in Gegenwart von 2-Methyl-5-äthylpyridin als Base und Vanadylacetylacetonat oder Vanadium-(III)-acetylacetonat als Katalysator durchgeführt.

Als Oxidationsmittel wird erfindungsgemäss Sauerstoff oder ein sauerstoffhaltiges Gas verwendet. Die Sauerstoffkonzentration ist nicht kritisch. Zweckmässigerweise betgrägt sie jedoch etwa 10-100 Vol.-%.Bei Verwendung eines sauerstoffhaltigen Gases ist die Selektivität der Reaktion im allgemeinen höher als bei Verwendung von reinem Sauerstoff. Als sauerstoffhaltiges Gas wird zweckmässig ein Gemisch von Sauerstoff und einem inerten Gas, wie Stickstoff, Argon und dergleichen, eingesetzt. Vorzugsweise wird die Oxidation mit einem sauerstoffhaltigen Gas, welches etwa 20-40 Vol-% Sauerstoff enthält, durchgeführt. Besonders bevorzugt ist die Verwendung von Luft als Oxidationsmittel.

Die Reaktionstemperatur ist nicht kritisch. Der optimale Temperaturbereich ist vom verwendeten Lösungsmittel abhängig. Im allgemeinen liegt er bei etwa 60 °C bis 90 °C (bzw. Rückflusstemperatur, sofern diese niedriger ist). Besonders bevorzugt ist ein Temperaturbereich von etwa 72 °C bis 83 °C, wenn Aethylenchlorid als Lösungsmittel verwendet wird.

Die Katalysatormenge ist nicht kritisch, und die Reaktion kann auch ohne Katalysator durchgeführt werden. Bei Verwendung der oben als bevorzugt genannten Katalysatoren wird jedoch mit Vorteil mindestens etwa 1 Mol-% Katalysator bezogen auf Retroionon eingesetzt.

Das erhaltene Reaktionsgemisch kann in einfacher Weise nach an sich bekannten Methoden aufgearbeitet werden. Beispielsweise kann durch Extraktion der Base mit Säure (z. B. Salzsäure) und Wasser und anschliessender Destillation und Umkristallisation des Rohproduktes 4-Oxo-β-ionon in guter Ausbeute und hoher Reinheit isoliert werden.

Die Erfindung wird durch die folgenden Beispiele weiter veranschaulicht.

Beispiel 1

In einem zylinderförmigen Glasreaktor mit Thermometer, Gaseinleitungsrohr mit Fritte und leistungsfähigem Kondenser wurden unter Argon 2,25 g Vanadylacetylacetonat in 900 ml Aethylenchlorid suspendiert. Die Suspension wurde mit 13,5 ml 2-Methyl-5-äthylpyridin versetzt und dann auf Rückflusstemperatur (⩾ 72 °C) erhitzt, wobei durch die Fritte ein Strom von 2 1/Min. Luft eingeleitet wurde. Sobald die Rückflusstemperatur erreicht war, wurden innert 2 Stunden 90,0 g Retroionon (enthaltend 85 % E-Isomer und 14 % Z-Isomer) zugetropft. Das Einleiten von Luft wurde bei 74-75 °C noch ca. 5,5 Stunden fortgesetzt, wobei 3 Stunden nach beendetem Zutropfen des Retroionons nochmals 0,56 g Vanadylacetylacetonat zum Reaktionsgemisch zugefügt wurden. Nach beendeter Reaktion wurde das Reaktionsgemisch am Rotationsverdampfer vom Lösungsmittel befreit. Der dunkelbraune Rückstand wurde in 1 Liter Cyclohexan aufgenommen und nacheinander mit 300 ml 2N Salzsäure, 300 ml gesättigter Natriumhydrogencarbonat-Lösung und zweimal mit je 500 ml Wasser extrahiert. Die wässrigen Phasen wurden nacheinander noch mit einer Portion von 1 Liter Cyclohexan extrahiert. Die organischen Phasen wurden vereinigt, über Magnesiumsulfat getrocknet und am Rotationsverdampfer eingedampft. Der erhaltene dunkelbraune Rückstand (94,3 g) enthielt gemäss Gaschromatographie mit internem Standard 82,7 % 4-Oxo-β-ionon (Ausbeute 80 %). Der Rückstand wurde in einer Hickmann-Apparatur bei 0,15-0,25 mbar und 120 °C Badtemperatur destilliert. Das Produkt wurde nach einem kleinen Vorlauf (1,1 g) in einer einzigen Fraktion gesammelt, wobei 80,8 g 86 %-iges 4-Oxo-β-ionon als hellgelbes, spontan erstarrendes Oel erhalten wurden (Ausbeute 72 %). 80,5 g des destillierten Produktes wurden unter Erwärmen in 20 ml Cyclohexan gelöst. Die Lösung wurde mit 60 ml Hexan versetzt, angeimpft und über Nacht in den Kühlschrank gestellt. Die Kristalle wurden abgenutscht, zweimal mit je 50 ml eiskaltem Hexan gewaschen und am Rotationsverdampfer im Wasserstrahlvakuum bei 40 °C bis zur Gewichtskonstanz (ca. 2 Stunden) getrocknet. Hierbei wurden 66,5 g 97,5%-iges 4-Oxo-β-ionon als weisse Kristalle mit Smp. 51-53,5 °C erhalten (Ausbeute 67 %).

Beispiel 2

In verschiedenen Versuchen wurden in einem Reagenzglas mit Kühlwasseranschluss jeweils 50 mg Vanadium-(III)-acetyl-acetonat, 2 ml Lösungsmittel [Versuch (f) ohne Lösungsmittel], 1 ml destilliertes Retroionon und 0,15 ml 2-Methyl-5-äthylpyridin auf 70 °C erwärmt, und dann wurde während 2,5 Stunden durch eine Fritte reiner Sauerstoff in das Gemisch eingeleitet. Die Reaktionsgemische wurden mit Wasser versetzt und mit Diäthyläther ausgeschüttelt. Die Ausbeuten an 4-Oxo-β-ionon wurden mittels Gaschromatographie (Flächenprozente) bestimmt. Die verwendeten Lösungsmittel und die Ausbeuten sind in Tabelle 1 zusammengestellt. Die Bedingungen sind nicht optimiert.

Tabelle 1

| Versuch | Lösungsmittel | Ausbeute an 4-Oxo-β-ionon |
|---|---|---|
| (a) | Aethylenchlorid | 76,2% |
| (b) | Chloroform | 55,3% |
| (c) | Chlorbenzol | 38,9% |
| (d) | Methyldiglykol | 55,4% |
| (e) | Methylcellosolve | 50,4% |
| (f) | ohne Lösungsmittel | 37,0% |

Beispiel 3

In mehreren Versuchen wurden jeweils in einem Reagenzglas 1 ml destilliertes Retroionon und 2 ml Dimethylformamid vorgelegt und mit 50 mg Katalysator und in den Versuchen (a)-(j) zusätzlich mit 0,15 ml 2-Methyl-5-äthylpyridin versetzt. Anschliessend wurde das Reagenzglas in ein 70 °C warmes, thermostatisierbares Wasserbad gestellt und während 2,5 Stunden durch eine Fritte Sauerstoff eingeleitet. Anschliessend wurde abkühlengelassen, mit 2 ml Wasser und 4 ml Cyclohexan versetzt und kurz geschüttelt. Die obere, organische Phase wurde mittels Gaschromatographie (Flächenprozente) analysiert. Die verwendeten Katalysatoren und die Ausbeuten an 4-Oxo-β-ionon sind in Tabelle 2 zusammengestellt. Die Bedingungen sind nicht optimiert.

Tabelle 2

| Versuch[1)] | Katalysator[2)] | Ausbeute an 4-Oxo-β-ionon |
|---|---|---|
| (a) | $Pd(acac)_2$ | 44,5% |
| (b) | $Cr(acac)_3$ | 60,7% |
| (c) | $Co(OOCCH_3)_2$ | 50,5% |
| (d) | $Co(acac)_2$ | 44,4% |
| (e) | $Co(acac)_3$ | 45,6% |
| (f) | $La(acac)_3$ | 47,6% |
| (g) | $MoO_2(acac)_2$ | 48,0% |
| (h) | $Ni(acac)_2$ | 50,1% |
| (i) | $V(acac)_3$ | 66,9% |
| (j) | ohne Katalysator | 40,8% |
| (k) | $Pd(acac)_2$ | 47,5% |
| (l) | $Cr(acac)_3$ | 43,8% |
| (m) | $Co(OOCCH_3)_2$ | 47,2% |
| (n) | $Co(acac)_2$ | 41,5% |
| (o) | $Co(acac)_3$ | 44,9% |
| (p) | $Ni(acac)_2$ | 48,1% |
| (q) | ohne Katalysator | 41,8% |

¹) Versuche (a)-(j) mit 0,15 ml 2-Methyl-5-äthylpyridin ; Versuche (k)-(q) ohne 2-Methyl-5-äthylpyridin

²) acac = Acetylacetonat

## Patentansprüche

1. Verfahren zur Herstellung von 4-Oxo-β-ionon der Formel

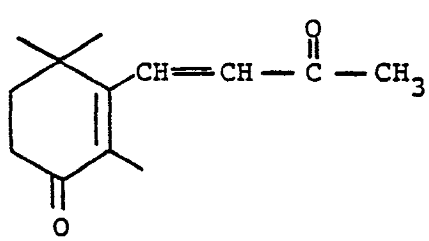

(I)

dadurch gekennzeichnet, dass man Retroionon der Formel

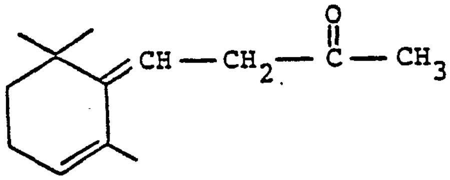

(II)

mit Sauerstoff oder einem sauerstoffhaltigen Gas oxidiert.

2. Verfahren nach Anspruch 1, dadurch gekennzeichnet, dass man die Oxidation in Gegenwart eines $C_1$-$C_{20}$-Carboxylates oder $C_2$-$C_{20}$-Enolates eines Uebergangsmetalls durchführt.

3. Verfahren nach Anspruch 2, dadurch gekennzeichnet, dass man die Oxidation in Gegenwart eines Acetates oder Acetylacetonates eines Uebergangsmetalls durchführt.

4. Verfahren nach Anspruch 3, dadurch gekennzeichnet, dass man die Oxidation in Gegenwart eines Acetates oder Acetylacetonates von Vanadium, Chrom, Kobalt, Nickel oder Molybdän, vorzugsweise in Gegenwart von Vanadylacetyl-acetonat oder Vanadium-(III)-acetylacetonat durchführt.

5. Verfahren nach einem der Ansprüche 1 bis 4, dadurch gekennzeichnet, dass man die Oxidation in Gegenwart von Pyridin oder einem alkylierten Pyridin, insbesondere 2-Methyl-5-äthylpyridin durchführt.

6. Verfahren nach Anspruch 5, dadurch gekennzeichnet, dass man die Oxidation in Gegenwart von Pyridin oder einem alkylierten Pyridin und einem Acetat oder Acetylacetonat von Vanadium, Chrom, Kobalt, Nickel oder Molybdän durchführt.

7. Verfahren nach einem der Ansprüche 1 bis 6, dadurch gekennzeichnet, dass man die Oxidation in einem chlorierten Alkan, einem Carbonsäureamid, einem Hydroxyäther oder einem chlorierten aromatischen Kohlenwasserstoff, vorzugsweise in Aethylenchlorid, durchführt.

8. Verfahren nach einem der Ansprüche 1 bis 7, dadurch gekennzeichnet, dass man die Oxidation in Aethylenchlorid in Gegenwart von 2-Methyl-5-äthylpyridin und Vanadylacetylacetonat oder Vanadium-(III)-acetylacetonat durchführt.

9. Verfahren nach einem der Ansprüche 1 bis 8, dadurch gekennzeichnet, dass man die Oxidation mit 10-100 %-igem Sauerstoff, vorzugsweise mit 20-40 %-igem Sauerstoff, durchführt.

10. Verfahren nach einem der Ansprüche 1 bis 9, dadurch gekennzeichnet, dass man die Oxidation bei einer Temperatur von 60-90 °C durchführt.

## Claims

1. A process for the manufacture of 4-oxo-β-ionone of the formula

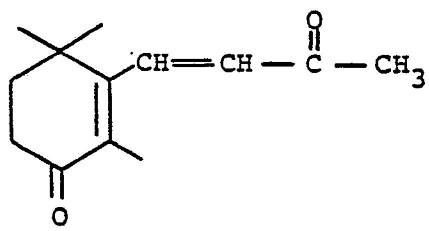

(I)

characterized by oxidizing retroionone of the formula

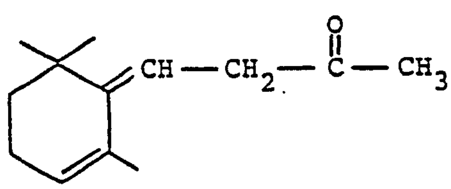

(II)

with oxygen or an oxygen-containing gas.

2. A process according to claim 1, characterized in that the oxidation is carried out in the presence of a $C_1$-$C_{20}$-carboxylate or $C_2$-$C_{20}$-enolate of a transition metal.

3. A process according to claim 2, characterized in that the oxidation is carried out in the presence of an acetate or acetylacetonate of a transition metal.

4. A process according to claim 3, characterized in that the oxidation is carried out in the presence of an acetate or acetylacetonate of vanadium, chromium, cobalt, nickel or molybdenum, preferably in the presence of vanadyl acetylacetonate or vanadium-(III) acetylacetonate.

5. A process according to any one of claims 1 to 4, characterized in that the oxidation is carried out in the presence of pyridine or an alkylated pyridine, especially 2-methyl-5-ethylpyridine.

6. A process according to claim 5, characterized in that the oxidation is carried out in the presence of pyridine or an alkylated pyridine and an acetate or acetylacetonate of vanadium, chromium, cobalt, nickel or molybdenum.

7. A process according to any one of claims 1 to 6, characterized in that the oxidation is carried out in a chlorinated alkane, a carboxylic acid amide, a hydroxyether or a chlorinated aromatic hydrocarbon, preferably in ethylene chloride.

8. A process according to any one of claims 1 to 7, characterized in that the oxidation is carried out in ethylene chloride in the presence of 2-methyl-5-ethylpyridine and vanadyl acetylacetonate or vanadium-(III) acetylacetonate.

9. A process according to any one of claims 1 to 8, characterized in that the oxidation is carried out with 10-100 % oxygen, preferably with 20-40 % oxygen.

10. A process according to any one of claims 1 to 9, characterized in that the oxidation is carried out at a temperature of 60-90 °C.

**Revendications**

1. Procédé de préparation de la 4-oxo-β-ionone de formule :

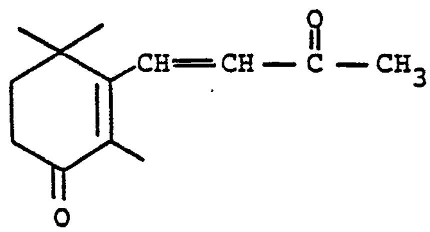

(I)

caractérisé en ce que l'on oxyde à l'aide d'oxygène ou d'un gaz contenant de l'oxygène la rétroionone de formule :

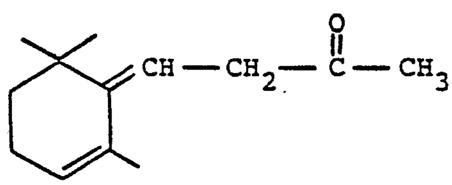

(II)

2. Procédé selon la revendication 1, caractérisé en ce que l'on effectue l'oxydation en présence d'un carboxylate en $C_1$-$C_{20}$ ou d'un énolate en $C_2$-$C_{20}$ d'un métal de transition.

3. Procédé selon la revendication 2, caractérisé en ce que l'on effectue l'oxydation en présence d'un acétate ou d'un acétylacétonate d'un métal de transition.

4. Procédé selon la revendication 3, caractérisé en ce que l'on effectue l'oxydation en présence d'un acétate ou d'un acétylacétonate du vanadium, du chrome, du cobalt, du nickel ou du molybdène, de préférence en présence de l'acétylacétonate de vanadyle ou de l'acétylacétonate de vanadium-III.

5. Procédé selon l'une des revendications 1 à 4, caractérisé en ce que l'on effectue l'oxydation en présence de pyridine ou d'une pyridine alkylée, en particulier la 2-méthyl-5-éthylpyridine.

6. Procédé selon la revendication 5, caractérisé en ce que l'on effectue l'oxydation en présence de pyridine ou d'une pyridine alkylée et d'un acétate ou d'un acétylacétonate du vanadium, du chrome, du cobalt, du nickel ou du molybdène.

7. Procédé selon l'une des revendications 1 à 6, caractérisé en ce que l'on effectue l'oxydation dans un alcane chloré, un carboxamide, un hydroxyéther ou un hydrocarbure aromatique chloré, de préférence dans le chlorure d'éthylène.

8. Procédé selon l'une des revendications 1 à 7, caractérisé en ce que l'on effectue l'oxydation dans le chlorure d'éthylène en présence de la 2-méthyl-5-éthylpyridine et de l'acétylacétonate de vanadyle ou de l'acétylacétonate de vanadium-III.

9. Procédé selon l'une des revendications 1 à 8, caractérisé en ce que l'on effectue l'oxydation avec de l'oxygène à une concentration de 10 à 100 %, de préférence de 20 à 40 %.

10. Procédé selon l'une des revendications 1 à 9, caractérisé en ce que l'on effectue l'oxydation à une température de 60 à 90 °C.